# EUROPEAN PATENT APPLICATION

(11) **EP 4 349 303 A2**
(43) Date of publication of application: **10.04.2024**
(21) Application number: 23197710.9
(22) Date of filing: 15.09.2023
(51) Int. Cl.: A61F 2/24

(54) **VALVE PROSTHESIS HAVING ROBUST PROSTHETIC VALVE AND METHOD**

(30) Priority: 04.10.2022 US 202263413079 P; 12.09.2023 US 202318465863
(71) Applicant: Medtronic Vascular, Inc., Santa Rosa, CA 95403 (US)
(72) Inventor: Cheshko, Tasha, Santa Ana, 92705 (US); Dorman, Kyle J., Santa Ana, 92705 (US); Kibria, Alkindi, Santa Ana, 92705 (US)
(74) Representative: Zimmermann & Partner Patentanwälte mbB

(57) **Abstract**

The techniques of this disclosure generally relate to a valve prosthesis including a one-piece molded valve having valve leaflets with free edges in a chevron down pattern. By forming the free edges in a chevron down pattern, pinwheeling of the prosthetic valve and the associated stress on the prosthetic valve is reduced.

## Description

### CROSS-REFERENCE TO RELATED APPLICATIONS

This application claims the benefit of U.S. Provisional Patent Application No. 16/413,079, filed October 4, 2022, the contents of which are incorporated by reference herein in their entirety.

### FIELD

The present technology is generally related to transcatheter heart valves.

### BACKGROUND

A human heart includes four heart valves that determine the pathway of blood flow through the heart: the mitral valve, the tricuspid valve, the aortic valve, and the pulmonary valve. The mitral and tricuspid valves are atrioventricular valves, which are between the atria and the ventricles, while the aortic and pulmonary valves are semilunar valves, which are in the arteries leaving the heart. Ideally, native leaflets of a heart valve move apart from each other when the valve is in an open position, and meet or "coapt" when the valve is in a closed position. Problems that may develop with valves include stenosis in which a valve does not open properly, and/or insufficiency or regurgitation in which a valve does not close properly. Stenosis and insufficiency may occur concomitantly in the same valve. The effects of valvular dysfunction vary, with regurgitation or backflow typically having relatively severe physiological consequences to the patient.

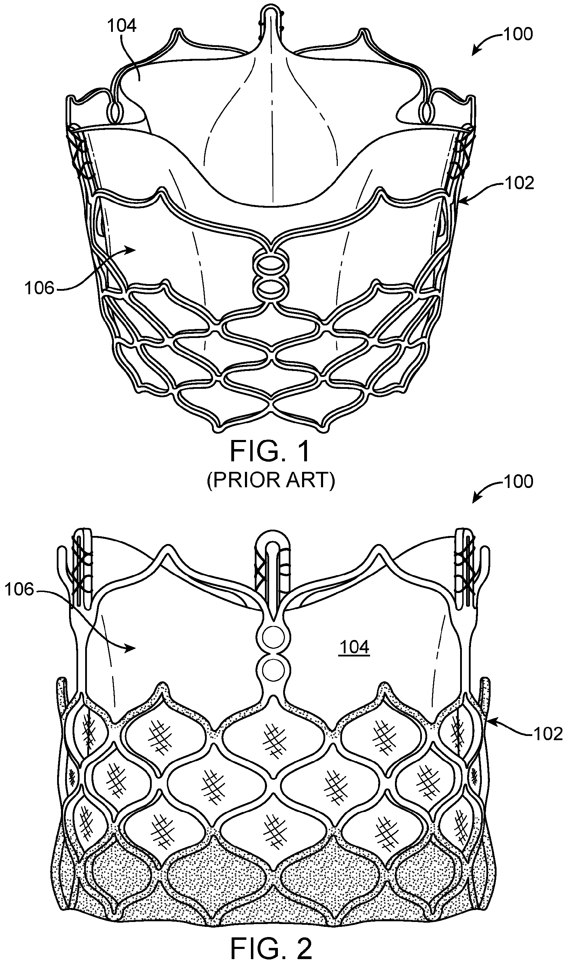

Recently, flexible prosthetic valves supported by stent structures that can be delivered percutaneously using a catheter-based delivery system have been developed for heart and venous valve replacement. FIG. 1 is a perspective view of a valve prosthesis 100 in accordance with the prior art. FIG. 2 is a side view of valve prosthesis 100 of FIG. 1 in accordance with the prior art. Referring to FIGS. 1 and 2, valve prosthesis 100 may include a balloon-expandable stent structure 102 with valve leaflets 104 attached to the interior of stent structure 102. Valve leaflets 104 may be parts of a 3D single piece prosthetic valve 106.

Valve prosthesis 100 can be reduced in diameter, by crimping onto a balloon catheter, and advanced through the venous or arterial vasculature. Once valve prosthesis 100 is positioned at the treatment site, for instance within an incompetent native valve, stent structure 102 may be expanded to hold valve prosthesis 100 firmly in place.

When designing a valve prosthesis such as valve prosthesis 100, valve-frame integration and frame mechanical performance often have competing needs or requirements. Embodiments hereof relate to an improved balloon-expandable transcatheter valve prosthesis configured to minimize tradeoffs between the above-described competing needs.

### SUMMARY

The techniques of this disclosure generally relate to a valve prosthesis including a one-piece molded valve having valve leaflets with free edges in a chevron down pattern. By forming the free edges in a chevron down pattern, pinwheeling of the prosthetic valve and the associated stress on the prosthetic valve is reduced.

In one aspect, the present disclosure provides a valve prosthesis including a one-piece molded valve. The one-piece molded valve includes valve leaflets defined by cusps and an attachment band abutting and proximal of the cusps. The attachment band follows a contour of the cusps. The attachment band is stitched to a stent structure and/or inner skirt of the valve prosthesis thus reducing undesirable movement of and the associated stress on the prosthetic valve.

In another aspect, the present disclosure provides a method including forming a one-piece molded valve by molding valve leaflets from a cylindrical piece of material, the valve leaflets being defined by cusps. The method further includes forming an attachment band abutting and proximal of the cusps by removing all of the cylindrical piece of material proximal of the attachment band.

In another aspect, the present disclosure provides a valve prosthesis including a stent structure, a prosthetic valve, and an inner skirt. The stent structure includes an inflow portion. The prosthetic valve is coupled to the stent structure, the prosthetic valve including valve leaflets defined by cusps and a valve inflow cylinder proximal of the cusps. The inner skirt is attached to an inner surface of the inflow portion and extends distally from the cusps to an outflow crown ring of the inflow portion. The valve inflow cylinder is directly attached to the inflow portion proximal of the inner skirt. By having the valve inflow cylinder nonoverlapping with the inner skirt, the profile of the prosthetic valve is reduced.

Further disclosed herein is a valve prosthesis including a one-piece molded valve having valve leaflets with free edges in a chevron down pattern, wherein, by forming the free edges in a chevron down pattern, pinwheeling of the prosthetic valve and the associated stress on the prosthetic valve is reduced.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a perspective view of a valve prosthesis in accordance with the prior art.
FIG. 2 is a side view of the valve prosthesis of FIG. 1 in accordance with the prior art.
FIG. 3 is a perspective view of a valve prosthesis in an expanded configuration in accordance with one embodiment.
FIG. 4 is an outflow end view of a prosthetic valve of the valve prosthesis of FIG. 3 in accordance with one embodiment.
FIG. 5 is a perspective view of the prosthetic valve of the valve prosthesis of FIG. 3 in accordance with one embodiment.
FIG. 6 is a side view of the valve prosthesis of FIG. 3 in accordance with one embodiment.
FIG. 7 is a laid flat plan view of a tissue coupon illustrating the prosthetic valve of the valve prosthesis of FIGS. 3-6 in accordance with one embodiment.
FIG. 8 is a perspective view of a prosthetic valve for use with the valve prosthesis of FIG. 3 in accordance with one embodiment.
FIG. 9 is a laid flat plan view of a tissue coupon illustrating the prosthetic valve of FIG. 8 during manufacturing in accordance with one embodiment.
FIG. 10 is a cross-sectional view of the prosthetic valve along the line X-X of FIG. 8 in accordance with one embodiment.
FIG. 11 is a cross-sectional view of the prosthetic valve along the line X-X of FIG. 8 in accordance with another embodiment.
FIG. 12 is a cross-sectional view of the prosthetic valve along the line X-X of FIG. 8 in accordance with yet another embodiment.
FIG. 13 is a cross-sectional view of the prosthetic valve along the line X-X of FIG. 8 in accordance with yet another embodiment.
FIG. 14 is a cross-sectional view of the prosthetic valve of FIG. 8 along the line X-X including a stent structure of the valve prosthesis of FIG. 6 and an outer skirt in accordance with one embodiment.
FIG. 15 is a side view of a valve prosthesis in accordance with one embodiment.
FIG. 16 is a perspective view of a prosthetic valve of the valve prosthesis of FIG. 15 in accordance with one embodiment.

### DETAILED DESCRIPTION

Specific embodiments are now described with reference to the figures, wherein like reference numbers indicate identical or functionally similar elements. The terms "distal" and "proximal", when used in the following description to refer to a native vessel, native valve, or a device to be implanted into a native vessel or native valve, such as a heart valve prosthesis, are with reference to the direction of blood flow. Thus, "distal" and "distally", also referred to as the "outflow" or "outflow direction", respectively, refer to positions in a downstream direction with respect to the direction of blood flow. The terms "proximal" and "proximally", also referred to as the "inflow" or "inflow direction", respectively, refer to positions in an upstream direction with respect to the direction of blood flow. Proximal is also sometimes referred to as inferior and distal is sometimes referred to as superior.

Although the description is in the context of treatment of an aortic heart valve, embodiments may also be used where it is deemed useful in other valved intraluminal sites that are not in the heart. For example, embodiments may be applied to other heart valves or venous valves as well.

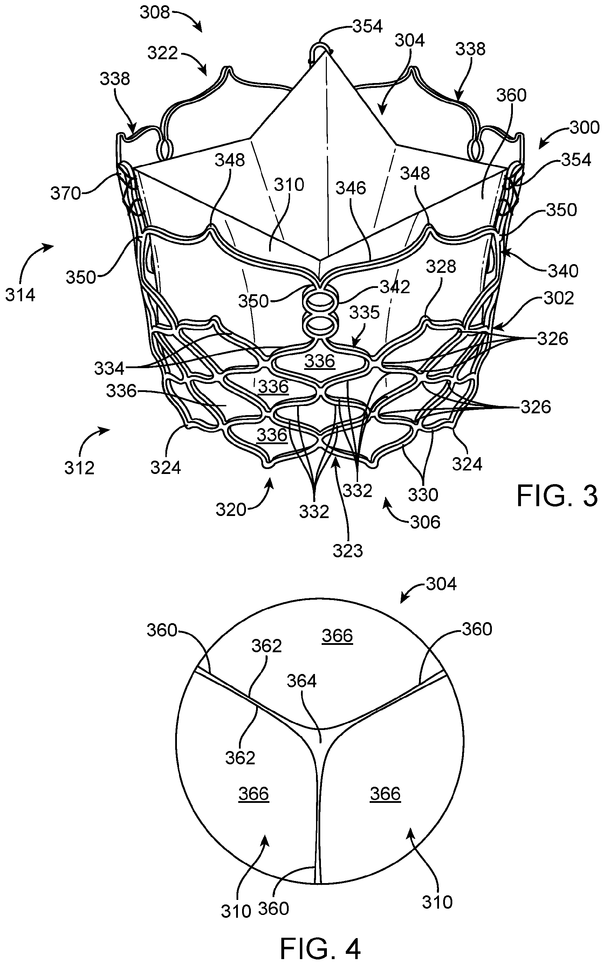

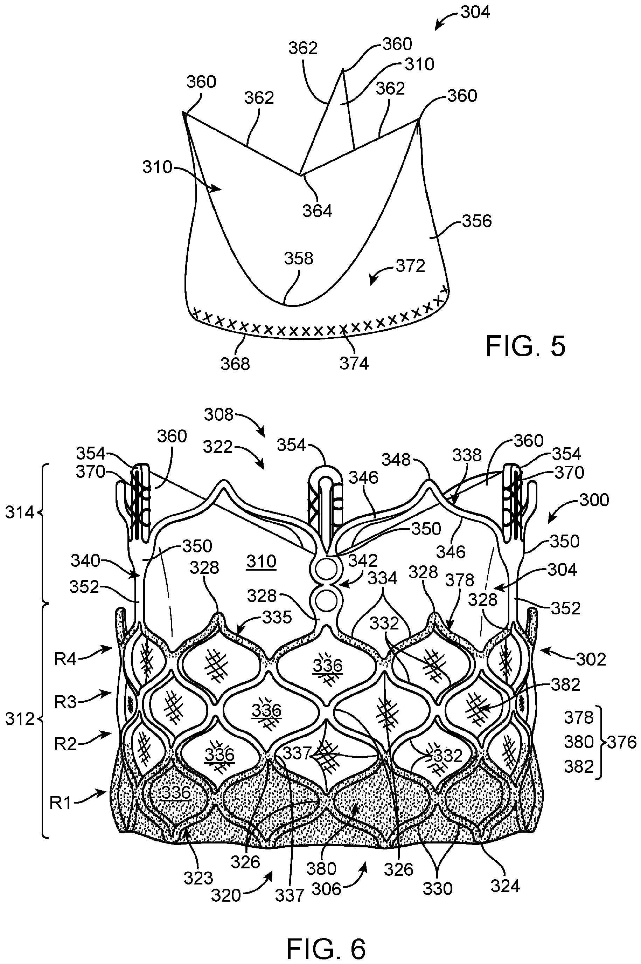

FIG. 3 is a side view of a transcatheter valve prosthesis 300 in the expanded configuration in accordance with one embodiment. FIG. 4 is an outflow end view of a prosthetic valve 304 of valve prosthesis 300 of FIG. 3 in accordance with one embodiment. FIG. 5 is a perspective view of prosthetic valve 304 of valve prosthesis 300 of FIG. 3 in accordance with one embodiment. FIG. 6 is a side view of valve prosthesis 300 of FIG. 3 in accordance with one embodiment. FIGS. 3, 6 illustrate prosthetic valve 304 in an open state whereas FIGS. 4-5 illustrates prosthetic valve 304 in a closed state.

Referring now to FIGS. 3-6 together, valve prosthesis 300 has a radially-expandable stent structure 302, sometimes called the frame, and prosthetic valve 304. Stent structure 302 is generally tubular, and is mechanically or balloon expandable, having a crimped configuration for delivery within a vasculature and an expanded configuration for deployment within a native heart valve. When valve prosthesis 300 is deployed within the valve annulus of a native heart valve, stent structure 302 of valve prosthesis 300 is configured to be radially expanded within native valve leaflets of the patient's defective valve, to thereby retain the native valve leaflets in a permanently open state. In embodiments hereof, valve prosthesis 300 is configured for replacement of an aortic valve such that an inflow end 306, i.e., the proximal end, of valve prosthesis 300 extends into and anchors within the aortic annulus of a patient's left ventricle, while an outflow end 308, i.e., the distal end, of valve prosthesis 300 is positioned within the aortic sinuses.

Stent structure 302 of valve prosthesis 300 may be a unitary frame or scaffold that supports prosthetic valve 304 including one or more valve leaflets 310 within the interior of stent structure 302. Prosthetic valve 304 is capable of blocking flow in one direction to regulate flow there-through via valve leaflets 310 that may form a bicuspid or tricuspid replacement valve. FIG. 4 illustrates that prosthetic valve 304 has three valve leaflets 310, i.e., prosthetic valve 304 has a tricuspid leaflet configuration, although a bicuspid leaflet configuration may be used in other embodiments. More particularly, as valve prosthesis 300 is configured for placement within a native aortic valve which typically has three leaflets, prosthetic valve 304 may include three valve leaflets 310. However, valve prosthesis 300 is not required to have the same number of leaflets as the native valve. If valve prosthesis 300 is alternatively configured for placement within a native valve having two leaflets such as the mitral valve, prosthetic valve 304 may include two or three valve leaflets 310.

Stent structure 302 is balloon-expandable. As such, stent structure 302 is made from a plastically deformable material such that when expanded by a dilatation balloon, stent structure 302 maintains its radially expanded configuration. Stent structure 302 may be formed from stainless steel such as 316L or other suitable metal, such as platinum iridium, cobalt chromium alloys such as MP35N or L605, or various types of polymers or other similar materials, including the materials coated with various surface deposits to improve clinical functionality. Stent structure 302 is configured to be rigid such that it does not deflect or move when subjected to in-vivo forces, or such that deflection or movement is minimized when subjected to in-vivo forces.

Stent structure 302 includes an inflow portion 312 and an outflow portion 314. Stent structure 302 is a tubular component defining a central lumen or passageway, and has an inflow end 320 and an outflow end 322. When expanded, a diameter of inflow end 320 of stent structure 302 is substantially the same as a diameter of outflow end 322 of stent structure 302 in one embodiment.

Inflow portion 312 extends distally from inflow end 320 of stent structure 302. Inflow portion 312 includes inflow crowns 324, central crowns 326, outflow crowns 328, inflow struts 330, central struts 332, and outflow struts 334. Inflow portion 312 further includes an inflow crown ring 323 defined by inflow crowns 324, inflow struts 330, and central crowns 326. Inflow portion further includes an outflow crown ring 335 defined by outflow crowns 328, outflow struts 334, and central crowns 326. Inflow portion 312 generally extends between inflow crown ring 323 and outflow crown ring 335.

In between inflow crown ring 323 and outflow crown ring 335, inflow portion cells 336, sometimes called side openings, are formed in rows, and more particularly, in four rows R1, R2, R3, and R4. Each row R1, R2, R3, R4 includes 12 inflow portion cells 336. Inflow portion cells 336 of the proximal most row R1 are defined by inflow crowns 324, inflow struts 330, central crowns 326, and central struts 332. Inflow portion cells 336 of the next most proximal rows R2, R3 are defined by central crowns 326 and central struts 332. Inflow portion cells 336 of the distal most row R4 are defined by outflow crowns 328, outflow struts 334, central crowns 326, and central struts 332. Generally, inflow portion cells 336 are diamond-shaped openings having the same or identical shaped, sometimes are called symmetric.

Generally, a crown is defined where two struts connect and a node is defined as a region where two crowns connect. Accordingly, inflow crowns 324 are defined where inflow struts 330 connect. Outflow crowns 328 are defined where outflow struts 334 connect. Central crowns 326 are defined where inflow struts 330 connect, where outflow struts 334 connect, and where central struts 332 connect. Central nodes 337 are defined where central crowns 326 connect.

Outflow portion 314 is formed proximate to outflow end 322 of stent structure 302 and between outflow end 322 and inflow portion 312. Outflow portion 314 includes an outflow crown ring 338, commissure posts 340, and non-commissure posts 342. Outflow portion 314 can be configured in a shape that forms a central lumen or passageway.

Outflow crown ring 338 includes outflow crown struts 346, superior crowns 348, and inferior crowns 350. Each outflow crown struts 346 is connected on one end to an adjacent outflow crown strut 346 at a superior crown 348 and on the opposite end to an adjacent outflow crown strut 346 at an inferior crown 350. Inferior crowns 350 are connected to either a commissure post 340 or a non-commissure post 342. More particularly, every other inferior crown 350 is connected to a commissure post 340 and every other inferior crown 350 is connected to a non-commissure post 342 in an alternating repeating arrangement.

Non-commissure posts 342, sometimes called axial frame members 342, extend longitudinally between and connect inferior crowns 350 of outflow crown ring 338 and outflow crowns 328 of inflow portion 312. In accordance with this embodiment, non-commissure posts 342 are shaped as a figure eight and can be used as markers for depth of implant as well as clocking of valve prosthesis 300. As used herein, longitudinally is in a direction parallel with the longitudinal axis, radially is perpendicular and in a radial direction from the longitudinal axis, and circumferentially is in a plane perpendicular to the longitudinal axis and in a direction along the circumference of valve prosthesis 300.

Commissure posts 340 extend longitudinally and include axial frame members 352 and trident posts 354. Axial frame members 352 extend longitudinally between and connect inferior crowns 350 of outflow crown ring 338 and outflow crowns 328 of inflow portion 312. Trident posts 354 extend in a cantilever fashion and in the outflow or distal direction from inferior crowns 350 of outflow crown ring 338. Axial frame members 352 and trident posts 354 are parallel with one another and are segments of commissure posts 340, which are linear members.

Prosthetic valve 304 is a one piece three dimensional (3D) molded structure in accordance with this embodiment. Illustratively, a single cylindrical piece is molded to form prosthetic valve 304. Accordingly, although various structures of prosthetic valve 304 are discussed below, prosthetic valve 304 is integral, i.e., formed from a single piece and not a plurality of separate pieces connected together.

Prosthetic valve 304 may be made of pericardial material; however, may instead be made of another material. Natural tissue for prosthetic valve 304 may be obtained from, for example, heart valves, aortic roots, aortic walls, aortic leaflets, pericardial tissue, such as pericardial patches, bypass grafts, blood vessels, intestinal submucosal tissue, umbilical tissue and the like from humans or animals. Synthetic materials suitable for use as prosthetic valve 304 include DACRON polyester commercially, other cloth materials, nylon blends, polymeric materials, and vacuum deposition nitinol fabricated materials. One polymeric material from which prosthetic valve 304 can be made is an ultra-high molecular weight polyethylene material. With certain materials, it may be desirable to coat one or both sides of prosthetic valve 304 with a material that will prevent or minimize overgrowth. It is further desirable that the material is durable and not subject to stretching, deforming, or fatigue.

Prosthetic valve 304 includes valve leaflets 310 and a valve inflow cylinder 356 proximal of valve leaflets 310. For example, valve inflow cylinder 356 is the remaining unmolded portion of the cylindrical material used to form prosthetic valve 304 and valve leaflets 310 are the molded portion.

Valve leaflets 310 are defined by cusps 358, commissures 360, and free edges 362. Adjoining pairs of valve leaflets 310 are attached to one another at their lateral ends to form commissures 360, with free edges 362 of valve leaflets 310 forming coaptation edges that meet in an area of coaptation 364. The region within cusps 358, commissures 360, and free edges 362 are sometimes referred to as a belly 366 of valve leaflets 310.

Valve inflow cylinder 356 has a cylindrical inflow end 368, sometimes called a nadir 368. Valve inflow cylinder 356 extends in the outflow direction from inflow end 368 as a cylinder to cusps 358, which form the outflow end of valve inflow cylinder 356.

Prosthetic valve 304 is disposed within and secured to at least trident posts 354 of commissure posts 340 of stent structure 302. In addition, prosthetic valve 304 may also be disposed within and secured to inflow portion 312 of stent structure 302. More particularly, commissures 360 are attached to trident posts 354, e.g., with commissure stitching 370. Further, a margin of attachment (MOA) 372 of valve inflow cylinder 356, e.g., a region directly adjacent inflow end 368, is attached to inflow portion 312, e.g., with MOA stitching 374. Although a particular location for MOA 372 adjacent inflow end 368 is illustrated in FIG. 5 and discussed above, in other embodiments, MOA 372 is in a different location. For example, MOA 372 is a parabolic MOA adjacent cusps 358. Other locations for MOA 372 are possible in yet other embodiments.

Valve prosthesis 300 further includes a skirt 376 which encloses or lines a portion of stent structure 302. Margin of attachment 372 of valve inflow cylinder 356 is sutured or otherwise securely and sealingly attached to the interior surface of skirt 376 and to inflow portion 312, e.g., with MOA stitching 374. Skirt 376 is not illustrated in FIG. 3 to allow visualization of stent structure 302.

Skirt 376 may enclose or line stent structure 302. Skirt 376 may be a natural or biological material such as pericardium or another membranous tissue such as intestinal submucosa. Alternatively, skirt 376 may be a low-porosity woven fabric, such as polyester, Dacron fabric, or PTFE. In one embodiment, skirt 376 may be a knit or woven polyester, such as a polyester or PTFE knit, which can be utilized when it is desired to provide a medium for tissue ingrowth and the ability for the fabric to stretch to conform to a curved surface. Polyester velour fabrics may alternatively be used, such as when it is desired to provide a medium for tissue ingrowth on one side and a smooth surface on the other side.

In accordance with this embodiment, skirt 376 includes an outflow end outer skirt 378, and inflow end outer skirt 380, and an inner skirt 382. Outflow end outer skirt 378 is located on the outer surface of outflow crowns 328 and outflow struts 334 of inflow portion 312. Inflow end outer skirt 380 is located on the outer surface of inflow crowns 324, inflow struts 330, central crowns 326, and central struts 332. More particularly, inflow end outer skirt 380 covers first row R1 of inflow portion cells 336. Inner skirt 382 is located on the inner surface of inflow portion 312 and extends between inflow crown ring 323 and outflow crown ring 335.

Delivery of valve prosthesis 300 may be accomplished via a percutaneous transfemoral approach or a transapical approach directly through the apex of the heart via a thoracotomy, or may be positioned within the desired area of the heart via different delivery methods known in the art for accessing heart valves. During delivery, valve prosthesis 300 remains compressed until it reaches a target diseased native heart valve, at which time a balloon of a delivery system is inflated in order to radially expand valve prosthesis 300 in situ. Valve prosthesis 300 is configured to be expanded within the native valve leaflets of the patient's defective valve, to thereby retain the native valve leaflets in a permanently open state. The delivery system is then removed and transcatheter valve prosthesis 300 remains deployed within the native target heart valve.

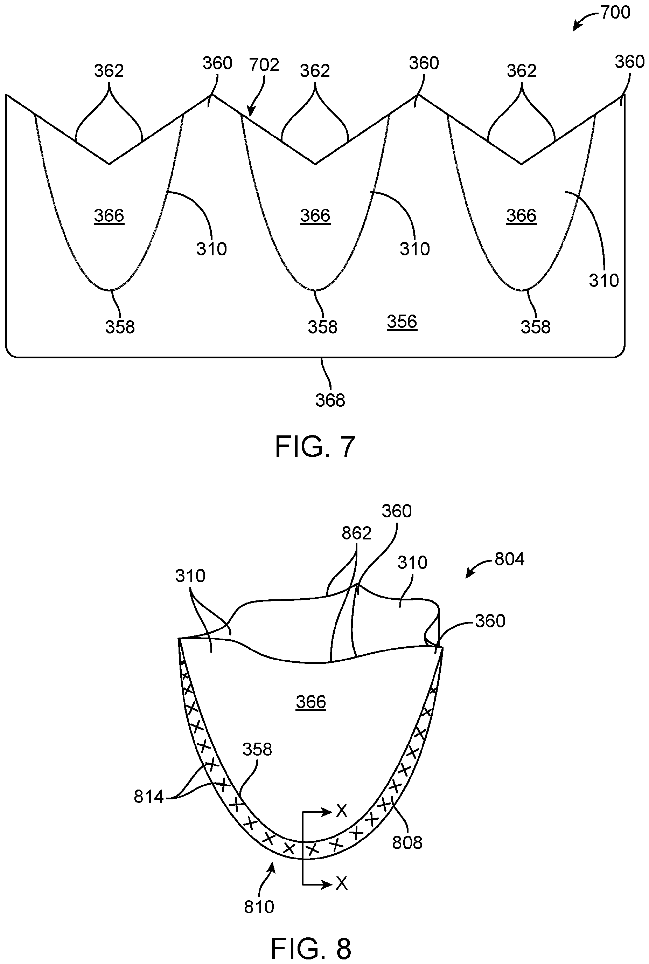

FIG. 7 is a laid flat plan view of a tissue coupon 700 illustrating prosthetic valve 304 of valve prosthesis 300 of FIGS. 3-6 in accordance with one embodiment. Tissue coupon 700 of FIG. 7 corresponds to prosthetic valve 304 of FIG. 5 cut longitudinally at a commissure 360. It is to be understood that tissue coupon 700 is set forth in FIG. 7 to facilitate understanding of the structure of prosthetic valve 304 but that prosthetic valve 304 is fabricated from a cylindrical piece of material and tissue coupon 700 would not exist during actual fabrication.

Referring now to FIGS. 5 and 7 together, free edges 362 of valve leaflets 310 have a chevron down pattern. A chevron down pattern means that each free edge 362 of each valve leaflet 310 is in the shape of a "V", with the tops of the "V" shape meeting at commissures 360.

More particularly, free edges 362 slant proximally (downward) from commissures 360 such that the area of coaptation 364 is proximal (downward in the view of FIGS. 5, 7) of commissures 360. As illustrated in FIG. 7, the superior edge 702 of tissue coupon 700 is chevron shaped, i.e., three "V" shapes connected together at commissures 360. Accordingly, valve leaflets 310 have less tissue as compared to a valve formed from a tissue coupon having a straight superior edge at the same height as commissures 360.

By removing tissue at free edges 362, sometimes called the free margin, to form a chevron down pattern, excess redundancy (tissue) is eliminated. By reducing tissue at free edges 362, e.g., with the chevron down pattern, pinwheeling is reduced. Pinwheeling is the undesirable back and forth rotation of prosthetic valve 304 within stent structure 302 during opening and closing of valve leaflets 310 typically associated with excess material of a prosthetic valve. By reducing pinwheeling, stress of prosthetic valve 304 during opening and closing is reduced.

FIG. 8 is a perspective view of a prosthetic valve 804 for use with valve prosthesis 300 of FIG. 3 in accordance with one embodiment. Prosthetic valve 804 of FIG. 8 is similar to prosthetic valve 304 of FIG. 5 and only the significant differences between prosthetic valve 804 and prosthetic valve 304 are discussed below. In prosthetic valve 804, free edges 862 are linear (straight) edges as compared to the chevron down pattern of free edges 362 of prosthetic valve 304.

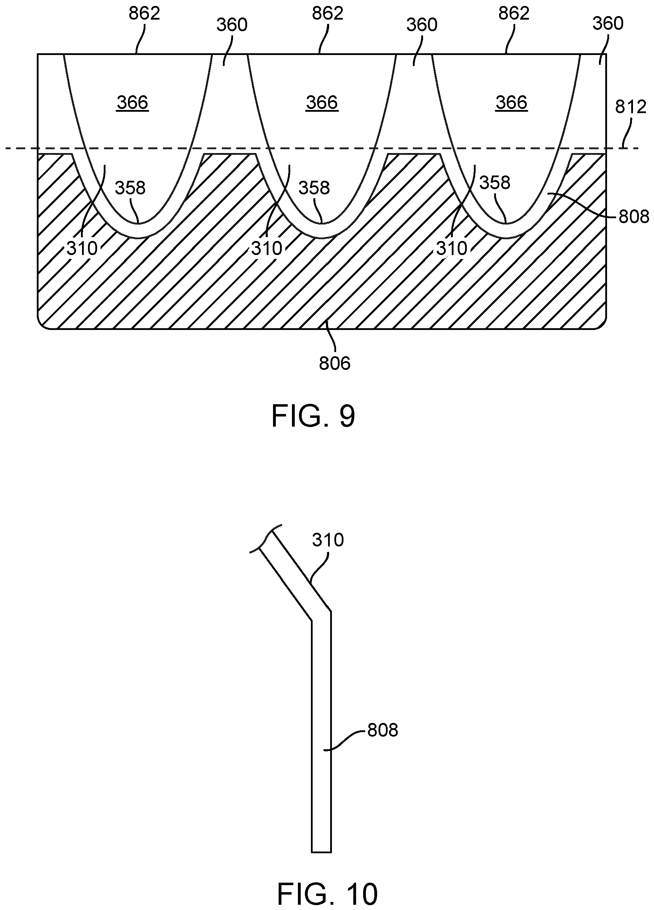

FIG. 9 is a laid flat plan view of a tissue coupon 900 illustrating prosthetic valve 804 of FIG. 8 during manufacturing in accordance with one embodiment. Tissue coupon 900 of FIG. 9 corresponds to prosthetic valve 804 of FIG. 8 cut longitudinally at a commissure 360 and during manufacturing of prosthetic valve 804. It is to be understood that tissue coupon 900 is set forth in FIG. 9 to facilitate understanding of the structure of prosthetic valve 804 but that prosthetic valve 804 is fabricated by 3D molding a cylindrical piece of material and tissue coupon 900 would not exist during actual fabrication.

Referring now to FIGS. 8 and 9 together, in accordance with this embodiment, all tissue 806 proximal of an attachment band 808 of prosthetic valve 804 is removed, e.g., by cutting. The removed tissue 806 is illustrated in FIG. 9 and not in FIG. 8 as the removed tissue 806 does not exist in the final prosthetic valve 804 illustrated in FIG. 8. By removing tissue 806, the profile of prosthetic valve 804 is reduced. Further, by removing tissue 806, prosthetic valve 804 has a parabolic shaped inflow end 810 that follows the contour of cusps 358, sometimes called the nadir of prosthetic valve 804.

Attachment band 808 is a strip of material that abuts and is just proximal of cusps 358 and an inferior coaptive area (ICA) 812. Inferior coaptive area 812 is the inferior point where free edges 862 of valve leaflets 310 meet in the area of coaptation 364. Attachment band 808 follows the contour of cusps 358 and defines inflow end 810 in a parabolic shape, i.e., inflow end 810 is in the shape of three parabolas and so is said to have a parabolic shape.

In one embodiment, attachment band 808 is at least 1 mm wide in the longitudinal direction to allow attachment band 808 to be attached to stent structure 302 and/or inner skirt 382 (stent structure 302 and inner skirt 382 are illustrated in FIG. 6). For example, attachment band 808 is stitched to stent structure 302 and/or inner skirt 382 by MOA stitching 814 as illustrated in FIG. 8. By stitching attachment band 808 to stent structure 302 and/or inner skirt 382, undesirable movement of and the associated stress on prosthetic valve 804 is reduced.

FIG. 10 is a cross-sectional view of prosthetic valve 804 along the line X-X of FIG. 8 in accordance with one embodiment. Referring now to FIGS. 8 and 10 together, attachment band 808 is a single layer of tissue that is integral with the tissue of valve leaflets 310. For example, as set forth above, prosthetic valve 804 is an integral 3D molded valve and thus attachment band 808 and valve leaflets 310 are portions of the tissue of the molded valve.

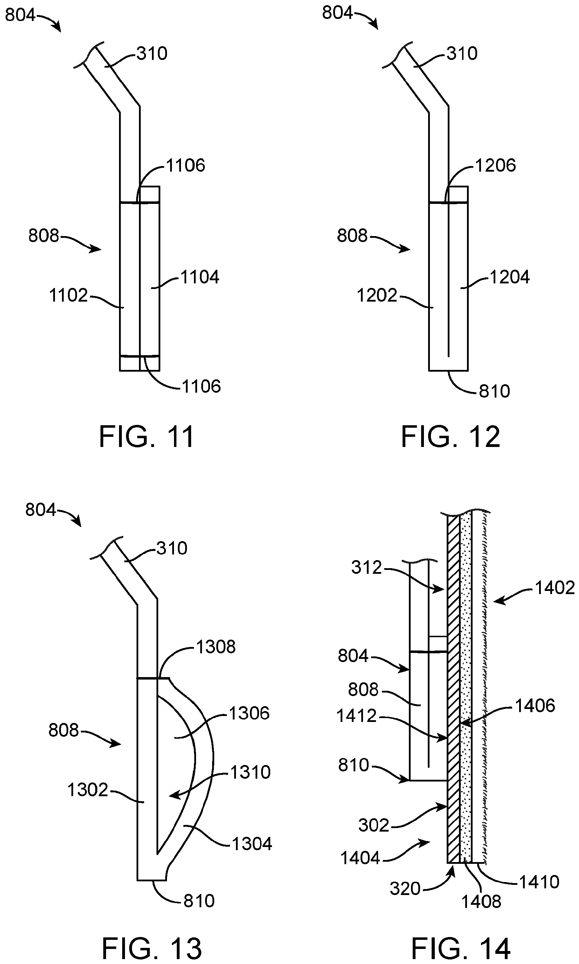

FIG. 11 is a cross-sectional view of prosthetic valve 804 along the line X-X of FIG. 8 in accordance with another embodiment. Referring now to FIGS. 8 and 11 together, attachment band 808 is a bilayer structure having a first tissue layer 1102 that is integral with the tissue of valve leaflets 310 and a second reinforcement layer 1104 attached to first tissue layer 1102 with stitching 1106. For example, as set forth above, prosthetic valve 804 is an integral 3D molded valve and first tissue layer 1102 and valve leaflets 310 are portions of the tissue of the molded valve.

Second reinforcement layer 1104 is formed of any of the materials listed above for prosthetic valve 304. Second reinforcement layer 1104 strengthens and reinforces first tissue layer 1102. Accordingly, second reinforcement layer 1104 strengthens and reinforces attachment band 808, which is stitched to stent structure 302 and/or inner skirt 382 by MOA stitching 814 as illustrated in FIG. 8.

FIG. 12 is a cross-sectional view of prosthetic valve 804 along the line X-X of FIG. 8 in accordance with yet another embodiment. Referring now to FIGS. 8 and 12 together, attachment band 808 is a bilayer structure having a first tissue layer 1202 and a second reinforcement layer 1204 that are both integral with the tissue of valve leaflets 310. More particularly, second reinforcement layer 1204 is folded from first tissue layer 1202 at inflow end 810 of prosthetic valve 804 and then stitched to first tissue layer 1202 by stitching 1206.

For example, as set forth above, prosthetic valve 804 is an integral 3D molded valve and first tissue layer 1202, second reinforcement layer 1204, and valve leaflets 310 are portions of the tissue of the molded valve.

Second reinforcement layer 1204 strengthens and reinforces first tissue layer 1202. Accordingly, second reinforcement layer 1204 strengthens and reinforces attachment band 808, which is stitched to stent structure 302 and/or inner skirt 382 by MOA stitching 814 as illustrated in FIG. 8.

FIG. 13 is a cross-sectional view of prosthetic valve 804 along the line X-X of FIG. 8 in accordance with yet another embodiment. Referring now to FIGS. 8 and 13 together, attachment band 808 includes a first tissue layer 1302, a second reinforcement layer 1304, a radiopaque filler 1306, and stitching 1308.

First tissue layer 1302 and second reinforcement layer 1304 are both integral with the tissue of valve leaflets 310. More particularly, second reinforcement layer 1304 is folded from first tissue layer 1302 at inflow end 810 of prosthetic valve 804 and then stitched to first tissue layer 1302 by stitching 1308. The space between first tissue layer 1302 and second reinforcement layer 1304 forms a pocket 1310, into which radiopaque filler 1306 is contained. Stitching 1308 seals pocket 1310 at the distal end.

For example, as set forth above, prosthetic valve 804 is an integral 3D molded valve and first tissue layer 1302, second reinforcement layer 1304, and valve leaflets 310 are portions of the tissue of the molded valve. Second reinforcement layer 1304 strengthens and reinforces first tissue layer 1302 while at the same time creating pocket 1310 into which radiopaque filler 1306 is contained. Radiopaque filler 1306 includes radiopaque material that allows visualization of attachment band 808. By visualizing attachment band 808, nadir placement for each valve leaflet 310 is readily identified.

FIG. 14 is a cross-sectional view of prosthetic valve 804 of FIGS. 8, 12 along the line X-X including stent structure 302 of valve prosthesis 300 of FIG. 6 and an outer skirt 1402 in accordance with one embodiment.

Referring now to FIGS. 6, 8, 12, and 14 together, as discussed above, inflow portion 312 of stent structure 302 is has a cylindrical shape with an inflow end 320. In contrast, prosthetic valve 804 has a parabolic shaped inflow end 810. Accordingly, prosthetic valve 804 does not cover inflow portion 312 all the way to inflow end 320. This uncovered portion 1404 of inflow portion 312 may cause leakage, e.g., paravalvular leakage (PVL).

To prevent the leakage, the entire outer surface 1406 of inflow portion 312 is covered with outer skirt 1402. In this embodiment, outer skirt 1402 is a bilayer structure including a smooth inner liner 1408 and a fuzzy outer layer 1410. Smooth inner liner 1408 is a robust liner and prevents, for example, calcification of the natural heart valve from penetrating through stent structure 302 and damaging prosthetic valve 804. Fuzzy outer layer 1410, e.g., a velour or other material having loose fibers, encourages tissue ingrowth and endothelization. By encouraging tissue ingrowth, fuzzy outer layer 1410 minimizes the chance of migration of the valve prosthesis.

In one embodiment, inner skirt 382 (illustrated in FIG. 6) is removed. Accordingly, as illustrated in FIG. 14, attachment band 808 is directly connected to an inner surface 1412 of inflow portion 312 of stent structure 302. Although the folded/pleated attachment band 808 of the embodiment of FIG. 12 is illustrated in FIG. 14 as an example, in other examples, attachment band 808 of the embodiments of FIGS. 10, 11, or 13 are directly attached to inner surface 1412 in a manner similar to that illustrated in FIG. 14.

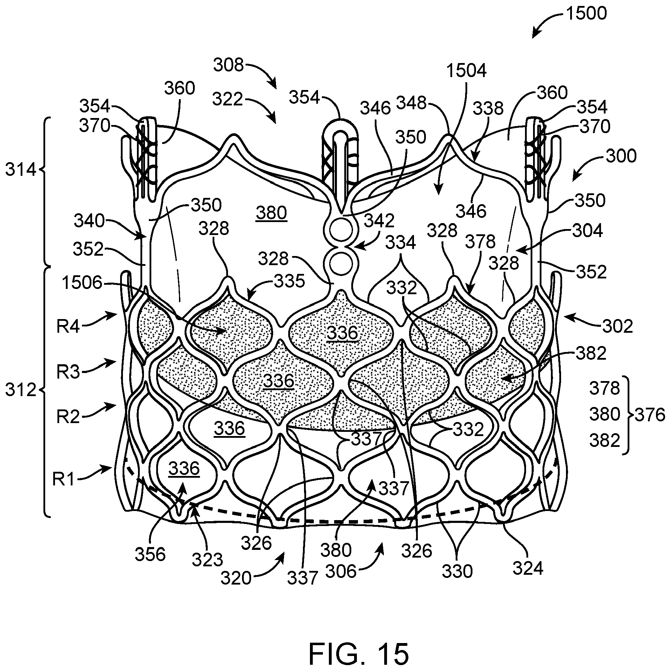

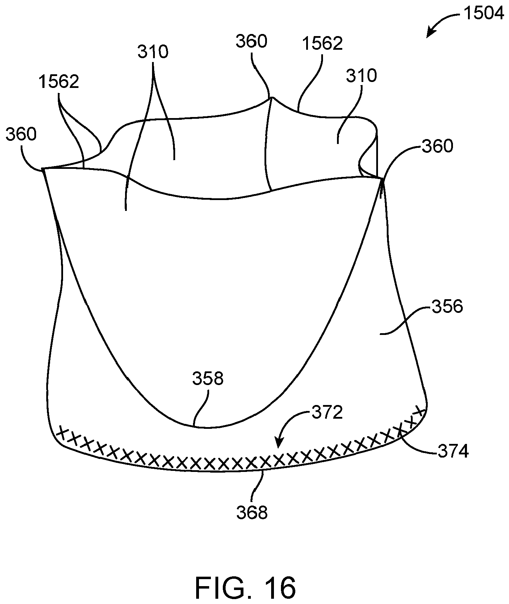

FIG. 15 is a side view of a valve prosthesis 1500 in accordance with one embodiment. FIG. 16 is a perspective view of a prosthetic valve 1504 of valve prosthesis 1500 of FIG. 15 in accordance with one embodiment. Valve prothesis 1500 and prosthetic valve 1504 of FIGS. 15 and 16 are similar to valve prosthesis 300 and prosthetic valve 304 of FIGS. 5-6 and only the significant differences are discussed below. In prosthetic valve 1504, free edges 1562 are linear (straight) edges as compared to the chevron down pattern of free edges 362 of prosthetic valve 304.

Referring now to FIGS. 15-16, an inner skirt 1506 extends distally from cusps 358 of prosthetic valve 1504 to outflow crown ring 335 of inflow portion 312 of stent structure 302. Inner skirt 1506 does not overlap valve inflow cylinder 356 of prosthetic valve 1504. Inner skirt 1506 is attached directly to the inner surface of inflow portion 312 of stent structure 302 to allow for a floating MOA/nadir (the region just proximal of cusps 358 in this embodiment) of prosthetic valve 1504.

Accordingly, inner skirt 1506 is provided between valve leaflets 310 and inflow portion 312 of stent structure 302 and thus protects valve leaflets 310 from damage caused by direct contact with inflow portion 312.

In contrast, valve inflow cylinder 356 of prosthetic valve 1504 is directly attached to inflow portion 312 of stent structure 302 proximal of inner skirt 1506. In other words, a proximal portion of the inner skirt between valve inflow cylinder 356 of prosthetic valve 1504 and inflow portion 312 of stent structure 302 has been eliminated in valve prosthesis 1500 as compared to valve prosthesis 300 of FIG. 6. By eliminating the proximal portion of the inner skirt and having valve inflow cylinder 356 be nonoverlapping with inner skirt 1506, the profile of prosthetic valve 1504 is reduced.

In FIG. 15, outer skirt(s) on the outer surface of inflow portion 312 are not illustrated to allow visualization of inner skirt 1506 and valve inflow cylinder 356. In one embodiment, prosthetic valve 1504 does not have an outer skirt on inflow portion 312. In other embodiments, prosthetic valve 1504 has one or more outer skirts on inflow portion 312, for example, similar to outer skirts 378, 380 of prosthetic valve 304 of FIG. 6.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

Further disclosed herein is the subject-matter of the following clauses:
1. A valve prosthesis comprising:
   a one-piece molded valve comprising:
   valve leaflets having free edges in a chevron down pattern.
2. The valve prosthesis of Clause 1 wherein the free edges have a "V" shape.
3. The valve prosthesis of Clause 2 wherein the one-piece molded valve further comprises commissures, tops of the "V" shape of each free edge meeting at the commissures.
4. The valve prosthesis of Clause 1 or of any of the preceding Clauses, wherein the one-piece molded valve further comprises commissures, the free edges slanting proximally from the commissures.
5. The valve prosthesis of Clause 1 or of any of Clauses 1-3, wherein the one-piece molded valve further comprises commissures, the free edges meeting in an area of coaptation that is proximal of the commissures.
6. A valve prosthesis comprising:
   a one-piece molded valve comprising:
   valve leaflets defined by cusps; and
   an attachment band abutting and proximal of the cusps, the attachment band following a contour of the cusps.
7. The valve prosthesis of Clause 6 wherein the one-piece molded valve further comprises a parabolic shaped inflow end defined by the attachment band.
8. The valve prosthesis of Clause 6 or of any of Clauses 6-7, wherein the attachment band is integral with the valve leaflets.
9. The valve prosthesis of Clause 6 or of any of Clauses 6-8, wherein the attachment band comprises a first layer that is integral with the valve leaflets and a second reinforcement layer attached to the first layer.
10. The valve prosthesis of Clause 6 or of any of Clauses 6-7, wherein the attachment band comprises a first layer and a second reinforcement layer that are integral with the valve leaflets, the second reinforcement layer being folded from the first layer at an inflow end of the one-piece molded valve.
11. The valve prosthesis of Clause 10 further comprising stitching attaching the second reinforcement layer to the first layer.
12. The valve prosthesis of Clause 10 wherein the first layer and the second reinforcement layer define a pocket, the valve prosthesis further comprising a radiopaque material in the pocket.
13. The valve prosthesis of Clause 6 or of any of Clauses 6-12, further comprising:
   a stent structure; and
   stitching attaching the attachment band to the stent structure.
14. The valve prosthesis of Clause 13 wherein the stent structure comprises an inflow portion, the valve prosthesis further comprising:
   an outer skirt covering an outer surface of the inflow portion.
15. The valve prosthesis of Clause 14 where the outer skirt comprises:
   an inner liner; and
   a fuzzy outer layer.
16. A valve prosthesis comprising:
   a stent structure comprising an inflow portion;
   a prosthetic valve coupled to the stent structure, the prosthetic valve comprising:
      valve leaflets defined by cusps; and
      a valve inflow cylinder proximal of the cusps; and
   an inner skirt attached to an inner surface of the inflow portion, the inner skirt extending distally from the cusps to an outflow crown ring of the inflow portion, the valve inflow cylinder being directly attached to the inflow portion proximal of the inner skirt.
17. The valve prosthesis of Clause 16 wherein the inner skirt is provided between the valve leaflets and the inflow portion.
18. The valve prosthesis of Clause 16 or of any of Clauses 16-17, wherein the inner skirt does not overlap the valve inflow cylinder.

## Claims

1. A valve prosthesis comprising:
a one-piece molded valve comprising:
valve leaflets having free edges in a chevron down pattern.

2. The valve prosthesis of Claim 1 wherein the free edges have a "V" shape.

3. The valve prosthesis of Claim 2 wherein the one-piece molded valve further comprises commissures, tops of the "V" shape of each free edge meeting at the commissures.

4. The valve prosthesis of any of the preceding Claims wherein the one-piece molded valve further comprises commissures, the free edges slanting proximally from the commissures, or wherein the one-piece molded valve further comprises commissures, the free edges meeting in an area of coaptation that is proximal of the commissures.

5. A valve prosthesis comprising:
a one-piece molded valve comprising:
valve leaflets defined by cusps; and
an attachment band abutting and proximal of the cusps, the attachment band following a contour of the cusps.

6. The valve prosthesis of Claim 5 wherein the one-piece molded valve further comprises a parabolic shaped inflow end defined by the attachment band.

7. The valve prosthesis of any of Claims 5-6 wherein the attachment band is integral with the valve leaflets, or wherein the attachment band comprises a first layer that is integral with the valve leaflets and a second reinforcement layer attached to the first layer.

8. The valve prosthesis of any of Claims 5-6 wherein the attachment band comprises a first layer and a second reinforcement layer that are integral with the valve leaflets, the second reinforcement layer being folded from the first layer at an inflow end of the one-piece molded valve.

9. The valve prosthesis of Claim 8 further comprising stitching attaching the second reinforcement layer to the first layer.

10. The valve prosthesis of Claim 8 wherein the first layer and the second reinforcement layer define a pocket, the valve prosthesis further comprising a radiopaque material in the pocket.

11. The valve prosthesis of any of Claims 5-10 further comprising:
a stent structure; and
stitching attaching the attachment band to the stent structure.

12. The valve prosthesis of Claim 11 wherein the stent structure comprises an inflow portion, the valve prosthesis further comprising:
an outer skirt covering an outer surface of the inflow portion.

13. The valve prosthesis of Claim 12 where the outer skirt comprises:
an inner liner; and
a fuzzy outer layer.

14. A valve prosthesis comprising:
a stent structure comprising an inflow portion;
a prosthetic valve coupled to the stent structure, the prosthetic valve comprising:
valve leaflets defined by cusps; and
a valve inflow cylinder proximal of the cusps; and
an inner skirt attached to an inner surface of the inflow portion, the inner skirt extending distally from the cusps to an outflow crown ring of the inflow portion, the valve inflow cylinder being directly attached to the inflow portion proximal of the inner skirt.

15. The valve prosthesis of Claim 14 wherein the inner skirt is provided between the valve leaflets and the inflow portion, and/or wherein the inner skirt does not overlap the valve inflow cylinder.
